# EUROPÄISCHE PATENTSCHRIFT

(11) **EP 1 274 421 B1**
(45) Veröffentlichungstag und Bekanntmachung des Hinweises auf die Patenterteilung: **02.08.2006**
(21) Anmeldenummer: 01935945.4
(22) Anmeldetag: 05.04.2001
(51) Int. Cl.: A61K 31/352, A61P 31/14

(54) **Verwendung von MEK-Inhibitoren zur Herstellung eines Arzneimittels gegen Negativstrang-RNA-Viren Infektionen**
Use of MEK-inhibitors for producing a medicament to treat negativstrang-RNA-viruses infections
Utilisation d'inhibiteurs de MEK pour la fabrication d'un médicament destiné à traiter les infections dues aux virus à RNA-strangnégatifs

(30) Priorität: 07.04.2000 DE 10017480
(43) Veröffentlichungstag der Anmeldung: 15.01.2003
(73) Patentinhaber: MedInnova Gesellschaft für medizinische Innovationen aus akademischer Forschung mbH, 35037 Marburg (DE)
(72) Erfinder: LUDWIG, Stephan, 97078 Würzburg (DE); PLESCHKA, Stephan, 35390 Giessen (DE)
(74) Vertreter: Jungblut, Bernhard Jakob
(86) Internationale Anmeldenummer: PCT/DE2001/001292
(87) Internationale Veröffentlichungsnummer: WO 2001/076570

(56) Entgegenhaltungen:
- WO-A-00/40237
- WO-A-98/37881
- WO-A-99/01421
- WO-A-99/01426
- DATABASE WPI Derwent Publications Ltd., London, GB; AN 2001-338036 XP002185544 & JP 2001 055376 A (WARNER LAMBERT CO), 27. Februar 2001 (2001-02-27)
- SMITH ET AL: "RAS-BINDING AND PHOSPHORYLATION BY HERPES SIMPLEX VIRUS TYPE 2 RR1 PK (ICP10)" JOURNAL OF VIROLOGY, Bd. 74, Nr. 22, November 2000 (2000-11), Seiten 10417-10429, XP002185543

## Beschreibung

Die vorliegende Erfindung basiert auf der erstmaligen Beobachtung, daß eine Infektion mit den intranukleär replizierenden Negativstrangviren insbesondere Influenza A virus und Borna Disease Virus (BDV) zur Aktivierung der Raf/MEK/ERK Kaskade führt und daß überraschenderweise die Inhibition dieser Kaskade insbesondere durch einen MEK Inhibitor die Replikation dieser virengruppe stark hemmt, ohne toxisch auf die Zellen zu wirken.

Eine verbesserte Therapie gegen Negativstrang RNA Viren, deren Vermehrung abhängig von der Aktivität der Raf/MEK/ERK Kaskade ist, richtet sich deshalb bevorzugt auf diesen Signalweg. Es wurde gefunden, daß dieser Signalweg durch verwendung eines nicht-toxischen pharmakologischen Inhibitors blockiert wird. Dieser nicht-toxische pharmakologische Inhibitor des Raf/MEK/ERK Signalweges ist erfindungsgemäß ein Kaskaden-Inhibitor, im besonderen ein MEK-Inhibitor.

### Stand der Technik

virusinfektionen stellen eine bedeutende Gesundheitsbedrohung für Mensch und Tier dar. Besonders Infektionen mit dem Influenza A Virus gehören immer noch zu den großen Seuchen der Menschheit und fordern Jahr für Jahr nicht nur eine vielzahl an Todesopfern, sondern sind auch gesamtwirtschaftlich, etwa durch krankheitsbedingte Arbeitsunfähigkeit ein immenser Kostenfaktor [12].

Von ebenfalls wichtiger wirtschaftlicher Bedeutung sind Infektionen mit dem Borna Disease Virus (BDV), das vor allem Pferde und Schafe befällt, jedoch auch schon beim Menschen isoliert wurde und hier in zusammenhang mit neurologischen Erkrankungen gebracht wird [3, 13].

Das Problem der Bekämpfung von RNA Viren ist die durch eine hohe Fehlerrate der viralen Polymerasen verursachte Wandlungsfähigkeit der Viren, die sowohl die Herstellung geeigneter Impfstoffe als auch das Entwickeln von antiviralen Substanzen sehr schwierig macht.

Es hat sich gezeigt, daß die Anwendung von antiviralen Substanzen, die direkt gegen Funktionen des Virus gerichtet sind, mutationsbedingt sehr schnell zur Selektion resistenter Varianten führt. Ein Beispiel hierfür ist das anti-Influenza Agenz Amantadin und dessen Derivate, welche gegen ein Transmembranprotein des Virus gerichtet sind und bereits binnen weniger Passagen zur Bildung resistenter Varianten führt. Auch die neuen anti-Influenza Therapeutika, die das Influenza-virale Oberflächenprotein Neuraminidase hemmen und unter dem Handelsnamen RELANZA von Glaxo Wellcome in Deutschland vertrieben werden, haben bereits resistente Varianten in Patienten erzeugt [10]. Die Hoffnungen, die in dieses Therapeutikum gelegt wurden konnten auch aus diesem Grunde nicht erfüllt werden.

Aufgrund ihrer meist kleinen Genome und damit begrenzter Kodierungskapazität für replikationsnotwendige Funktionen, sind alle Viren in starkem Maße auf Funktionen ihrer Wirtszellen angewiesen. Durch Einflußnahme auf solche zelluläre Funktionen, die für die virale Replikation notwendig sind, ist es möglich, die Virusreplikation in der infizierten Zelle negativ zu beeinträchtigen. Hierbei besteht für das Virus nicht die Möglichkeit, durch Anpassung die fehlende zelluläre Funktion zu ersetzen. Ein Entweichen vor dem Selektionsdruck durch Mutation ist hier nicht möglich. Dies konnte am Beispiel des Influenza A Virus mit relativ unspezifischen Hemmstoffen gegen zelluläre Kinasen und Methyltransferasen bereits gezeigt werden [18].

Nachteil speziell dieser Hemmstoffe ist, daß sie relativ unspezifisch und breit wirken und ihre zellulären Angriffspunkte nur schlecht definiert sind. Sie sind daher als Therapeutika ungeeignet. Hier liegt das Problem: Es gibt bis heute keine Hemmstoffe zellulärer Enzyme mit definiertem Angriffspunkt in der Zelle, die sowohl selektiv an dieser Stelle wirken, ohne toxisch für die Zelle zu sein, als auch die virale Replikation im besonderen von RNA Viren, wie Borna Viren oder Influenza A Viren hemmen.

In Bezug auf die zellulären Prozesse, welche nach Virusinfektion induziert werden, findet man, daß eine Vielzahl von DNA und RNA Viren in der infizierten Wirtszelle einen definierten Signaltransduktionsweg, die sogenannte Raf/MEK/ERK Kinase Kaskade aktivieren [2, 4, 14, 17].

Diese Kinase Kaskade gehört zu den wichtigsten Signalwegen in der Zelle und spielt eine bedeutende Rolle in Proliferations- und Differenzierungsprozessen.

Wachstumsfaktor induzierte Signale werden dabei durch sukzessive Phosphorylierung von der Serin/Threonin Kinase Raf auf die Dual-spezifische Kinase MEK (MAP Kinase Kinase/ERK Kinase) und schließlich auf die Kinase ERK (extracellular signal regulated kinase) übertragen. Während man als Kinasesubstrat von Raf nur MEK kennt und für MEK als einzige Substrate die ERK Isoformen identifiziert wurden, kann ERK eine ganze Reihe an Substraten phosphorylieren. Hierzu gehören beispielsweise die Phosphorylierung von Transkriptionsfaktoren, was zu einer direkten Veränderung der zellulären Genexpression führt [5, 15, 20].

Die Untersuchung der Rolle dieses Signalweges in zellulären Entscheidungsprozessen hat zur Identifizierung mehrerer pharmakologischer Inhibitoren geführt, welche den Signalweg unter anderem auf der Ebene von MEK, also am 'Flaschenhals' der Kaskade hemmen [1, 5, 7, 9].

Der MEK Inhibitor PD98059 (2-2'-amino-3'-methoxyphenyl)-oxanaphtalen-4-on) [7] hemmt die Aktivierung von MEK durch die Kinase Raf.

Der MEK Inhibitor U0126 (1,4-Diamino-2,3-dicyano-1, 4-bis[2-aminophenylthio]butadien) wurde als Substanz beschrie) ben, die partiell die Aktivierung AP-1 abhängiger Genexpression [9] und die Proliferation von T Zellen hemmt [6].

Im Gegensatz zu PD98059 hemmt U0126 nicht nur die MEK Aktivierung sondern auch die Aktivität der Kinase selbst [8].

Schließlich wurde der MEK Inhibitor PD184352 beschrieben 5 (2-(2-chlor-4-jod-phenylamino)-N-cyclopropylmethoxy-3,4-difluor-benzamid) [19], welcher bei oraler Gabe im Mausmodell effizient das wachstum von Kolonkarzinomen hemmen konnte, ohne erkennbare Zeichen von Toxizität bis zu einer kumulierten Dosis von 6g/kg Körpergewicht zu zeigen.

Die Dokumente WO 99/01426 A, WO 99/01421 A, WO 98/37881 A und WO 00/40237 A offenbaren verschiedene MEK-Inhibitoren und deren Verwendungen. Erkrankungen durch Infektionen mit Negativstrang-RNA-viren sind nicht angesprochen.

### Aufgabe der Erfindung

Der Erfindung lag die Aufgabe zugrunde, Substanzen zur Anwendung in der vorbeugung oder Therapie gegen intranukleär replizierende Negativstrang-RNA-Viren vorzusehen, die nicht direkt gegen Funktionen des virus gerichtet sind, sondern selektiv ein zelluläres Enzym hemmen und über diese selektive Wirkung die virale Replikation von Viren.

Es wurde nun überraschenderweise gefunden, daß diese Aufgabe durch ein einen erfindungsgemäß verwendeten MEK-Inhibitor enthaltendes Arzneimittel gelöst wird.

Ein erfindungsgemäß verwendeter MEK-Inhibitor, ist eine Substanz, die dadurch charakterisiert ist, daß sie in einem "Kaskaden Assay für Inhibitoren des Raf/MEK/ERK Kinase Signalweges" die Signalkaskade in vitro hemmt und in einem "in vivo MEK und MAP Kinase Assay" die Signalkaskade in vivo hemmt.

### Kaskaden Assay für Inhibitoren des Raf/MEK/ERK Kinase SignalWeges

Bei diesem Kaskaden Assay wird die Wirkung von Inhibitoren auf den Raf/MEK/ERK Kinase Signalweg durch Kinasevermittelten Einbau von radioaktivem 32P in das Myelin Basic Protein (MBP) in Anwesenheit eines 6xHistidin-Fusionsproteins von ERK (His-ERK) und eines Glutathione-S-transferase Fusionsproteins von MEK (GST-MEK) gemessen.

Das Reaktionsgemisch enthält die rekombinanten Proteine in einem Puffer aus 20mM HEPES, pH7.4, 10mM MgC12, 1mM MnC12, 1mM EGTA, und 50mM 32P-gamma-ATP in einem Gesamtvolumen von 100µl. Die Reaktion läuft 15 min bei 30°C und wird durch Zugabe von 20µl 5x Laemmli Puffer abgestoppt. Die radioaktiv markierten Proteine werden mittels SDS-PAGE aufgetrennt und mit Hilfe eines Phosphoimagers sichtbar gemacht. Kaskaden-Inhibitoren werden in einer Konzentration von 5-20µM auf ihre inhibierende Fähigkeit in diesem Assay getestet. Um zu unterscheiden, ob eine Verbindung in diesem Assay ein MEK oder ERK Inhibitor ist, werden die Substanzen in einem zweiten experimentellen Ansatz mit MBP und His-ERK unter den obigen Reaktionsbedingungen in Abwesenheit von GST-MEK getestet. Eine Verbindung die im ersten Ansatz wirksam ist und im zweiten Ansatz keine Wirkung zeigt, ist ein MEK Inhibitor. Eine Substanz die im zweiten Ansatz wirkt und im ersten Ansatz keine Wirkung zeigt ist ein ERK Inhibitor. Eine Substanz die in keinem der beiden Ansätze des ersten Assays wirkt, jedoch im folgenden in vivo MEK und MAP Kinase Assay eine Wirkung zeigt ist ein Raf-Inhibitor. Alle beschriebenen Inhibitoren sind erfindungsgemäß Kaskaden-Inhibitoren.

### In vivo MEK und MAP Kinase Assay

Zellen werden in 10 cm Zellkulturschalen ausgesät und wachsen bis 80% Konfluenz in Zellkulturmedium mit 10% fötalem Kälberserum. Den Zellen wird für 8-12h das Serum entzogen. Danach erfolgt Zugabe der Kaskaden-Inhibitoren, insbesondere der MEK-Inhibitoren 30 min. vor mitogener Stimulation der Zellen, beispielsweise mit 100ng/ml TPA oder 100ng/ml PDGF. Nach 10 min Inkubation mit den mitogenen Stimuli werden die Zellen mit PBS gewaschen und in Triton-Lyse Puffer lysiert (20mM Tris pH 7.4, 50mM Na-β-Glycerophosphat, 20mM Na-Pyrophosphat, 137mM NaCl, 10% (v/v) Glycerin, 1% (v/v) Triton X-100, 2mM EDTA, 1mM Pefabloc, 1mM Na-Vanadate, 5mM Benzamidin, 5µg/ml Aprotinin, 5µg/ml Leupeptin). Aus diesen Zellysaten wird endogene MEK mit einem MEK-spezifischen Antiserum immunpräzipitiert und in einem Immunkomplex Kinaseassay in Anwesenheit von 32P-gamma-ATP, 0.1mM ATP und rekombinanter kinaseinaktiver His-ERK K>M als Substratprotein bei 30°C für 15 min in einem Puffer aus 10mM MgC12, 25mM β-Glycerolphosphat, 25mM HEPES pH 7.5, 5mM Benzamidin, 0.5mM DTT und 1mM Na-Vanadat inkubiert. Parallel wird aus dem gleichen Lysat endogene ERK mit einem spezifischen ERK-Antiserum immunpräzipitiert und mit gereinigtem MBP unter den gleichen Bedingungen wie MEK inkubiert. Die Proteine werden auf einem SDS-PAGE Gel aufgetrennt und mit Hilfe eines Phosphoimagers visualisiert. Ein Kaskaden-Inhibitor, insbesondere ein MEK-Inhibitor wirkt in diesem Assay sowohl hemmend auf die MEK Aktivierung, messbar an der Phosphorylierung von His-ERK K>M, als auch auf die ERK Aktivierung, messbar an der Phosphorylierung von MBP.

Die erfindungsgemäße Verwendung der MEK-Inhibitoren bezieht sich insbesondere auf folgende Substanzen:
a) 2-(2-Amino-3-methoxyphenyl)-4-oxo-4H-(1)benzopyran (wie auch beschrieben in WO 98/37881)
b) 1,4-Diamino-2,3-dicyano-1,4-bis[2-aminophenylthio]butadien (Kurzbezeichnung U0126)
c) 2-(2-chlor-4-jod-phenylamino)-N-cyclopropylmethoxy-3,4-difluorbenzamid (Kurzbezeichnung PD18453)
d) 2-(2'-amino-3'-methoxyphenyl)-oxanaphthalen-4-on (Kurzbezeichnung PD98059)
e) Substanzen gekennzeichnet dadurch, daß sie als erfindungsgemäße Kaskaden-Inhibitoren wirken und insbesondere aus den chemischen Stoffklassen der Butadien-Derivate oder Flaven-Derivate oder Benzamid-Derivate stammen
f) alle als Kaskaden-Inhibitoren, insbesondere MEK-Inhibitoren wirkende Derivate der genannten verbindungen
g) weitere als der Kaskaden-Inhibitoren, insbesondere MEK-Inhibitoren wirkende Substanzen (Vorstufen, Salze oder "Prodrugs" im Sinne von [11, 16) der genannten Verbindungen oder ihrer Derivate deren Wirksamkeit im Kaskaden Assay für Inhibitoren des Raf/MEK/ERK Kinase Signalweges oder im "in vivo MEK und MAP Kinase Assay" belegt ist.

Die Erfindung betrifft die Verwendung dieser Substanzen als Arzneimittel an Patienten, die mit einem Negativstrang-RNA-Virus, speziell einem intranukleär replizierenden Negativstrang-RNA Virus, beispielsweise einem Influenza A Virus oder einem Borna Disease Virus infiziert sind.

In einer anderen Form der erfindungsgemäßen Verwendung wird vorgeschlagen, Arzneimittel mit diesen Substanzen in der Prävention einer Infektion mit einem Negativstrarrg-RNA-Virus, speziell einem intranukleär replizierenden Negativstrang-RNA Virus, beispielsweise einem Influenza A Virus oder einem Borna Disease Virus einzusetzen.

Der Begriff Patient bezieht sich dabei gleichermaßen auf Menschen und Wirbeltiere. Damit können die Arzneimittel in der Human- und veterindrmedizin verwendet werden. Die therapeutisch wirksamen Substanzen der vorliegenden Erfindung werden den Patienten, als Teil einer pharmazeutisch akzeptablen Komposition entweder oral, rektal, parenteral intravenös, intramuskulär oder subkutan, intracisternal, intravaginal, intraperitoneal, intravasculär, lokal (Puder, Salbe oder Tropfen) oder in Sprayform verabreicht.
Pharmazeutisch akzeptable Kompositionen können die Modifikationen als Salze, Ester, Amide und "Prodrugs" beinhalten, sofern sie nach zuverlässiger medizinischer Beurteilung keine übermäßige Toxizität, Irritationen oder allergische Reaktionen am Patienten auslösen.

Der Terminus "Prodrug" bezieht sich auf Verbindungen, die zur Verbesserung der Aufnahme transformiert werden, wie beispielsweise durch Hydrolyse im Blut. Eine eingehende Diskussion ist bei [11] und [16] gegeben.

Dosierungsformen für die örtliche Administration der Verbindung dieser Erfindung schließen Salben, Puder, Sprays oder Inhalationsmittel ein. Die aktive Komponente wird unter sterilen Bedingungen, mit einem physiologisch akzeptablen Trägerstoff und möglichen Preservativen, Puffern oder Treibmitteln, je nach Bedarf, vermischt.

### Beispiele

Das Ausführungsbeispiel 1 zeigt exemplarisch am Beispiel des MEK-Inhibitors U0126, daß mit steigender Konzentration des MEK-Inhibitors U0126 im Zellkulturmedium die Anzahl der neu gebildeten, infektiösen Influenza A Viruspartikel signifikant reduziert ist:

Für die Vermehrung von Influenza A Viren werden permissive, eukaryontische Zellkulturen (Madine darby canine kidney (MDCK)-Zellen), in parallelen Ansätzen mit gleicher Zellzahl, nach allgemein üblichen Methoden für die Zellkultur, mit einer physiologischen Salzlösung gewaschen und mit einer gleichen Menge des infektiösen Influenza A Virusstammes WSN-HK (Reassortante mit sieben Gensegmenten von Influenza Stamm A/WSN/33 und dem NA-Gen von Influenza Stamm A/HK/8/68), in einem Verhältnis von 0,0025 infektiöse Viruspartikel pro Zelle für eine Stunde bei Raumtemperatur, infiziert.

30 min vor der Infektion werden die MDCK-Zellen in geeignetem Zellkulturmedium, welches in unterschiedlichen Konzentrationen mit dem MEK-Inhibitor U0126 versetzt ist (0µM, 30µM, 40µM, 50µM gelöst in DSMO) bei 37°C und 5% CO2-Konzentration, inkubiert. Als Lösungsmittelkontrolle werden MDCK-Zellen mit Zellkulturmedium inkubiert, welches mit entsprechenden unterschiedlichen Mengen an DMSO versetzt ist. Während der Infektion wird dem Inokulum der MEK-Inhibitor U0126 bzw. als Lösungsmittel DMSO in den entsprechenden Konzentrationen zugegeben.

Anschließend wird das Inokulum entfernt und die infizierten Zellen in geeignetem Zellkulturmedium, welches in unterschiedlichen Konzentrationen mit dem MEK-Inhibitor U0126 versetzt sind (0µM, 30µM, 40µM, 50µM gelöst in DMSO), für 48h, bei 37°C und 5% CO₂-Konzentration, inkubiert. Als Lösungsmittelkontrolle werden infizierte MDCK-Zellen mit Zellkulturmedium inkubiert, welches mit entsprechenden unterschiedlichen Mengen an DMSO versetzt sind. 24 Stunden nach der Infektion werden 200µl des Mediumüberstandes entnommen und das gleiche Volumen an Inhibitor- bzw. DMSO-haltigem Zellkulturmedium dem Mediumüberstand wieder zugegeben. Nach 48 Stunden wird erneut eine Probe entnommen. Die Zellkulturüberstände der jeweiligen Proben für den 24 und den 48 Stunden Wert werden nach üblichen virologischen Methoden auf die Menge an haemagglutinierenden Einheiten (HA-Titer), welche die Gesamtproduktion an Viruspartikeln darstellt, und auf die Menge an neu gebildeten infektiösen Viruspartikeln (Plaque-Assay auf MDCK-Zellen) hin untersucht.

Im Ergebnis kann in einem solchen experimentellen Ansatz festgestellt werden, daß es bei zunehmender Konzentration des MEK-Inhibitors U0126 im Zellkulturmedium zu einer signifikanten Reduktion (ca. 80% bei 50µM U0126) der Anzahl von neu gebildeten, infektiösen Viruspartikeln, im Vergleich zum Kontrollansatz ohne MEK-Inhibitor U0126 bzw. den Lösungsmittelkontrollen kommt. Die makroskopische Untersuchung von MDCK Zellen, welche mit entsprechenden Konzentrationen an DMSO, bzw. in DMSO gelöstem MEK-Inhibitor U0126 behandelt wurden, als auch eine Zytotoxizitätsuntersuchung mittels Propidiumjodidfärbung, zeigt, daß weder Lösungsmittel noch Inhibitor einen signifikanten zytotoxischen Effekt auf die Zellen haben.

Das Ausführungsbeispiel 2 zeigt, daß mit steigender Konzentration des MEK-Inhibitors U0126 im Zellkulturmedium auch die Anzahl der neu gebildeten, infektiösen Borna Disease Viruspartikel signifikant reduziert wird:
Inhibitor-vorbehandelte Zellen werden mit BDV infiziert und die Ausbreitung der Infektion in einer indirekten Immunfluoreszenz gegen das virale Nucleoprotein beobachtet. Bei einer einmaligen Gabe von 25µM MEK Inhibitor (U0126) sind nach einer Kultivierungszeit von 7 Tagen keine Virus-Foci sichtbar, sondern nur einzelne infizierte Zellen. Bei einer Gabe von 12,5 µM Kinase Inhibitor (U0126) ist der Effekt nicht mehr deutlich und bei einer Gabe von 6 µM Kinase Inhibitor (U0126) ist kein Unterschied der Virus-Foci im Vergleich zu unbehandelten, infizierten Kontrollzellen zu sehen. Der Inhibitor wirkt somit dosisabhängig auf der Ebene der Virusreplikation.

Die inhibitorische Wirkung des MEK Inhibitors U0126 in den aufgezeigten Anwendungen zeigt, daß Kaskaden-Inhibitoren, insbesondere MEK Inhibitoren als antivirale Agentien gegen Influenza- und Borna Viren im speziellen aber auch gegen andere RNA und DNA Viren, bei denen eine Abhängigkeit der viralen Vermehrung von der Aktivität der Raf/MEK/ERK Kaskade besteht, angewendet werden können. Der Signalweg ist dabei erfindungsgemäß Ziel der antiviralen Therapie und wird bevorzugt durch Anwendung eines nicht-toxischen pharmakologischen Kaskaden-Inhibitors, insbesondere eines MEK Inhibitors, gehemmt.

### Bezugszeichenliste Literatur

[1] Alessi, D. R., Cuenda, A., Cohen, P., Dudley, D. T., and Saltiel, A. R. (1995). PD 098059 is a specific inhibitor of the activation of mitogen-activated protein kinase kinase in vitro and in vivo J. Biol. Chem. 270, 27489-27494.
[2] Benn, J., Su, F., Doria, M., and Schneider, R. J. (1996). Hepatitis B virus HBx protein induces transcription factor AP-1 by activation of extracellular signal-regulated and c-Jun N-terminal mitogen-activated protein kinases. J. Virol. 70, 4978-4985.
[3] Bode, L., Zimmermann, W., Ferszt, R., Steinbach, F., and Ludwig, H. (1995). Borna disease virus genome transcribed and expressed in psychiatric patients [see comments]. Nat Med 1, 232-6.
[4] Bruder, J. T., and Kovesdi, 1. (1997). Adenovirus infection stimulates the Raf/MAPK signaling pathway and induces interleukin-8 expression. J. Virol. 71, 398-404.
[5] Cohen, P. (1997). The search for physiological substrates of MAP and SAP kinases in mammalian cells. Trends in Cell Biol. 7, 353-361.
[6] DeSilva, D. R., Jones, E. A., Favata, M. F., Jaffee, B. D., Magolda, R. L., Trzaskos, J. M., and Scherle, P. A. (1998). Inhibition of mitogen-activated protein kinase kinase blocks T cell proliferation but does not induce or prevent anergy. J. Immunol. 160, 4175-4181.
[7] Dudley, D. T., Pang, L., Decker, S. J., Bridges, A. J., and Saltiel, A. R. (1995). A synthetic inhibitor of the mitogen-activated protein kinase cascade. Proc. Natl. Acad. Sci. U S A 92, 7686-7689.
[8] Duncia, J. V., Santella, J. B. r., Higley, C. A., Pitts, W. J., Wityak, J., Frietze, W. E., Rankin, F. W., Sun, J. H., Earl, R. A., Tabaka, A. C., Teleha, C. A., Blom, K. F., Favata, M. F., Manos, E. J., Daulerio, A. J., Stradley, D. A., Horiuchi, K., Copeland, R. A., Scherle, P. A., Trzaskos, J. M., Magolda, R. L., Trainor, G. L., Wexler, R. R., Hobbs, F. W., and Olson, R. E. (1998). MEK inhibitors: the chemistry and biological activity of U0126, its analogs, and cyclization products. Bioorg Med Chem Lett 8, 2839-44.
[9] Favata, M. F., Horiuchi, K. Y., Manos, E. J., Daulerio, A. J., Stradley, D. A., Feeser, W. S., Van Dyk, D. E., Pitts, W. J., Earl, R. A., Hobbs, F., Copeland, R. A., Magolda, R. L., Scherle, P. A., and Trzaskos, J. M. (1998). Identification of a novel inhibitor of mitogen-activated protein kinase kinase. J. Biol. Chem. 273, 18623-18632.
[10] Gubareva, L. V., Matrosovich, M. N., Brenner, M. K., Bethell, R. C., and Webster, R. G. (1998). Evidence for zanamivir resistance in an immunocompromised child infected with influenza B virus. J Infect Dis 178, 1257-62.
[11] Higuchi, T., and Stella, V. (1987). Prodrugs as novel delivery systems. In A.C.S. Symposium Series.
[12] Lamb, R. A., and Krug, R. M. (1996). Orthomyxoviridae: The viruses and their replication. In Fields Virology, B. N. e. a. Fields, ed. (Philadelphia: Lippincott-Raven Publishers), pp. 1353-1395.
[13] Planz, O., Rentzsch, C., Batra, A., Winkler, T., Buttner, M., Rziha, H. J., and Stitz, L. (1999). Pathogenesis of borna disease virus: granulocyte fractions of psychiatric patients harbor infectious virus in the absence of antiviral antibodies. J Virol 73, 6251-6.
[14] Popik, W., and Pitha, P. M. (1998). Early activation of mitogen-activated protein kinase kinase, extracellular signal-regulated kinase, p38 mitogen-activated protein kinase, and c-Jun N-terminal kinase in response to binding of simian immunodeficiency virus to Jurkat T cells expressing CCR5 receptor. Virology 252, 210-217.
[15] Robinson, M. J., and Cobb, M. H. (1997). Mitogen-activated protein kinase pathways. Curr. Opin. Cell Biol. 9, 180-186.
[16] Roche, E. B. E. (1987). Bioreversible Carriers in Drug Design, E. B. Roche, ed.: American Pharmaceutical Association and Pergamon Press.
[17] Rodems, S. M., and Spector, D. H. (1998). Extracellular signal-regulated kinase activity is sustained early during human cytomegalovirus infection. J. Virol. 72, 9173-9180.
[18] Scholtissek, C., and Muller, K. (1991). Failure to obtain drug-resistant variants of influenza virus after treatment with inhibiting doses of 3-deazaadenosine and H7. Arch Virol. 119, 111-118.
[19] Sebolt-Leopold, J. S., Dudley, D. T., Herrera, R., Van Becelaere, K., Wiland, A., Gowan, R. C., Tecle, H., Barrett, S. D., Bridges, A., Przybranowski, S., Leopold, W. R., and Saltiel, A. R. (1999). Blockade of the MAP kinase pathway suppresses growth of colon tumors in vivo. Nature Med. 5, 810-816.
[20] Treisman, R. (1996). Regulation of transcription by MAP kinase cascades. Curr. Opin. Cell Biol. 8, 205-215.

## Patentansprüche

1. Verwendung eines MEK-Inhibitors zur Herstellung eines Arzneimittels zur Vorbeugung oder Behandlung von Infektionen durch Negativstrang-RNA-Viren bei Mensch und Tier.

2. Verwendung nach Anspruch 1 zur Behandlung von Infektionen durch intranukleär replizierende Negativstrang-RNA-Viren.

3. Verwendung nach Anspruch 1 zur Behandlung von Infektionen durch Influenza- und Bornea-Viren.

4. Verwendung nach Anspruch 1, wobei der MEK-Inhibitor ausgewählt ist aus der Gruppe bestehend aus "2-(2-Amino-3-methoxyphenyl)-4-oxo-4H-(1)benzopyran, U0126, PD184352 und PD98059".

## Claims

1. Use of a MEK-inhibitor for the production of a drug for the prevention or treatment of infections by negative strand RNA viruses in man and animal.

2. Use according to claim 1 for the treatment of infections by intranuclear-replicating negative strand RNA viruses.

3. Use according to claim 1 for the treatment of infections by influenza and Borna viruses.

4. Use according to claim 1, wherein the MEK-inhibitor is selected from the group consisting of "2-(2-Amino-3-methoxyphenyl)-4-oxo-4H-(1)benzopyran, U0126, PD184352 and PD98059".

## Revendications

1. Utilisation d'un inhibiteur de MEK pour la production d'un médicament pour la prévention ou le traitement d'infections par les virus à ARN à brin négatif chez l'homme et l'animal.

2. Utilisation selon la revendication 1 pour le traitement d'infections par les virus à ARN à brin négatif se répliquant intranucléairement.

3. Utilisation selon la revendication 1 pour le traitement d'infections par les virus de la grippe et les bornavirus.

4. Utilisation selon la revendication 1, dans laquelle l'inhibiteur de MEK est choisi à partir du groupe se composant de "2-(2-amino-3-méthoxyphényl)-4-oxo-4H-(1)benzopyran, U0126, PD184352 et PD 98059".
